# EUROPÄISCHE PATENTANMELDUNG

(11) **EP 0 876 829 A2**
(43) Veröffentlichungstag der Anmeldung: **11.11.1998**
(21) Anmeldenummer: 98106264.9
(22) Anmeldetag: 06.04.1998
(51) Int. Cl.: A61N 5/06

(54) **Gerät für die künstliche Bräunung der Haut**

(30) Priorität: 08.08.1997 IT TV970117; 24.04.1997 IT TV970049
(71) Anmelder: Sun Club S.r.l., 31100 Treviso (IT)
(72) Erfinder: Röhrich, Helmut Gustav, 31100 Trevisco (IT)
(74) Vertreter: Glawe, Delfs, Moll & Partner

(57) **Zusammenfassung**

Gegenstand der Erfindung ist ein kabinenförmiges Gerät für die künstliche Bräunung der Haut. Die Strahlungsintensität in der Nähe des Bodens dieses Geräts wird dadurch verbessert, daß in Bodennähe Reflexionsflächen (25) vorgesehen sind.

## Beschreibung

Gegenstand der vorliegenden Erfindung ist ein Gerät für die künstliche Bräunung der Haut, das aus wenigstens einer Kabine besteht, an deren Wänden Strahlungspaneele vorgesehen sind.

Der Erfindung liegt die Beobachtung zugrunde, daß der Bräunungseffekt im unteren Bereich der Beine mitunter zu wünschen übrigläßt. Das gilt auch dann, wenn in diesem Bereich an den Wänden Strahlungspaneele vorgesehen sind. Sie führt dies darauf zurück, daß die Oberfläche des Bodens einen beträchtlichen Teil der bräunenden Strahlung absorbiert und dadurch die Intensität des bräunenden Strahlungsanteils in diesem Bereich verringert ist.

Dieser Nachteil wird erfindungsgemäß dadurch aufgehoben oder verringert, daß in der Nähe des Kabinenbodens wenigstens ein die Strahlung reflektierendes Element angeordnet ist, das die zum Boden hin gerichtete Strahlung ganz oder teilweise zum Bräunungsbereich zurückwirft. Unter Beachtung der geometrischen Bedingungen, die durch dieses Ziel gesetzt sind, kann die Anordnung der reflektierenden Flächen beliebig gewählt werden. Eine besonders einfache und deshalb bevorzugte Ausführungsform ist die, daß das reflektierende Mittel von der Oberfläche des Bodens selbst gebildet ist. Beispielsweise kann ein runder Sockel der Kabine mit einer metallischen Oberfläche versehen sein, die vorzugsweise aus Aluminium besteht.

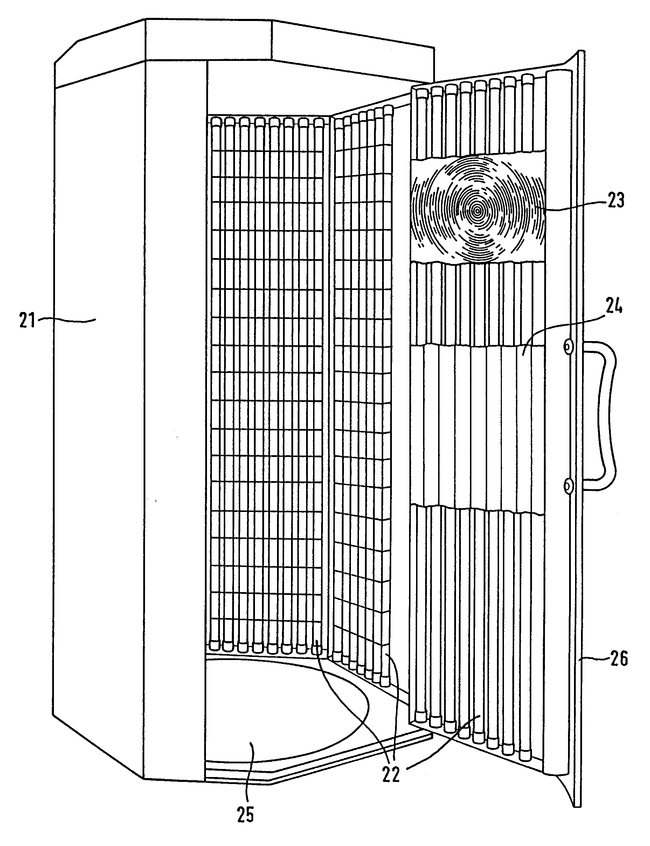

Weitere Eigenschaften und Vorteile der Erfindung ergeben sich aus der folgenden Beschreibung einer bevorzugten, jedoch nicht ausschließlichen Ausführung, die in den beigefügten Zeichnungen dargestellt ist. Darin zeigt die einzige Figur ein kabinenförmiges Gerät, das für die Aufnahme von einem oder mehreren senkrechten Strahlungspaneelen eingerichtet ist.

Es handelt sich um eine Kabine 21, in welche sich der Nutzer stellt oder setzt und die eine oder mehrere senkrechte Strahlungspaneele 22 für Strahlung enthält, die in einem im wesentlichen kontinuierlichen Spektrum durch Hochdrucklampen und/oder in einem im wesentlichen linienförmigen Spektrum durch Niederdrucklampen erzeugt wird. Vor den Paneelen 22 können ein oder mehrere Mittel angeordnet werden für die Deformierung und/oder die Konzentration und/oder die Verstärkung der Strahlen, vorzugsweise in Form einer oder mehrerer Fresnel-Linsen 23 oder Zylinderlinsen 24. Die Länge dieser Linsen kann gleich der Höhe der Kabine oder der senkrechten Paneele oder kürzer sein.

In Höhe des Kabinenbodens ist ein die Strahlung reflektierendes Element vorgesehen, nämlich in Form eines runden Podestes 25 mit metallischer Oberfläche vorzugsweise aus Aluminium. Dadurch wird eine verbesserte Bräunung der Beine erreicht.

Diese Ausführung erleichtert es, die gesetzten Ziele zu erreichen, und sie ermöglicht dem Nutzer, eine optimale und einheitliche Bräunung in kürzerer Zeit zu erreichen als mit herkömmlichen Methoden.

Vorzugsweise läßt sich die Kabine rings um den Benutzer herum schließen. In dem dargestellten Beispiel ist die Kabine für diesen Zweck mit einer Tür 26 ausgerüstet. Unbedingt erforderlich ist dies aber nicht.

Es ist vorteilhaft, wenn die Paneele 22 senkrecht angeordnet sind. Sie können aber auch gegenüber der Vertikalrichtung ein wenig geneigt sein. In dem Beispiel sind die Paneele bis zum Boden der Kabine geführt. Es ist aber auch möglich, sie in Abstand über dem Boden enden zu lassen. Die dadurch im Unterschenkelbereich verringerte Strahlungsintensität kann durch geeignet angeordnete Reflexionsflächen zumindest teilweise kompensiert werden.

## Patentansprüche

1. Gerät für die künstliche Bräunung der Haut, das aus wenigstens einer Kabine besteht, an deren Wänden Strahlungspaneele vorgesehen sind, dadurch gekennzeichnet, daß in der Nähe des Kabinenbodens wenigstens ein die Strahlung reflektierendes Element (25) angeordnet ist.

2. Gerät nach Anspruch 1, dadurch gekennzeichnet, daß das reflektierende Mittel die Oberfläche des Bodens bildet.

3. Gerät nach Anspruch 1 oder 2, dadurch gekennzeichnet, daß das reflektierende Mittel aus einem metallischen runden Sockel besteht, vorzugsweise aus Aluminium.
